# EUROPEAN PATENT SPECIFICATION

(11) **EP 2 020 902 B1**
(45) Date of publication and mention of the grant of the patent: **20.07.2011**
(21) Application number: 07747472.4
(22) Date of filing: 29.05.2007
(51) Int. Cl.: A61B 1/267, A61B 5/00

(54) **OPTICAL TRIGGERING SYSTEM FOR STROBOSCOPE SYSTEM, AND METHOD**
OPTISCHES TRIGGERSYSTEM FÜR STROBOSKOP, UND VERFAHREN
SYSTÈME DE DÉCLENCHEMENT OPTIQUE POUR UN SYSTÈME STROBOSCOPIQUE, ET PROCÉDÉ

(30) Priority: 26.05.2006 EP 06076120
(43) Date of publication of application: 11.02.2009
(73) Proprietor: Cymo B.V., 9723 JC Groningen (NL)
(72) Inventor: QIU, Qingjun, NL-9722 BH Groningen (NL); SCHUTTE, Harm Kornelis, NL-9745 DM Groningen (NL); VAN GEEST, Lambertus Karel, NL-9313 TH Leutingewolde (NL)
(74) Representative: Altenburg, Bernardus Stephanus Franciscus
(86) International application number: PCT/NL2007/050249
(87) International publication number: WO 2007/139381

(56) References cited:
- US-A1- 2003 139 666
- VALASKOVIC G A ET AL: "Automated orthogonal control system for electrospray ionization" JOURNAL OF THE AMERICAN SOCIETY FOR MASS SPECTROMETRY, ELSEVIER SCIENCE INC, US, vol. 15, no. 8, August 2004 (2004-08), pages 1201-1215, XP004523219 ISSN: 1044-0305

## Description

### FIELD OF THE INVENTION

The present invention relates to a triggering system for a stroboscope system, and to a method for generating triggering signals. The invention further relates to a stroboscope system, and to a method for stroboscopy. The invention further relates to information obtainable by a triggering system or a stroboscope system. The invention also relates to a laryngoscope.

### BACKGROUND OF THE INVENTION

Historically, a convenient method of getting a slow motion effect from high-speed vibratory movements which are too fast to be observed directly by the human eye, and hence appear only as a blur, is using the stroboscopic phenomenon. Most people are acquainted with this phenomenon. If a regular vibratory object is illuminated by short light flashes of the same frequency as the vibrations, the object will appear immobile. Likewise, if it is illuminated by flashes of a somewhat lower or higher frequency than the vibrations it appears to move slowly forwards or backwards. The frequency of the observed motion is determined by the difference between the vibratory frequency of the object and that of the flashes.

For instance, the vibratory movements of human vocal folds during phonation are too fast to be observed by human eye. In order to allow an observation of the vocal folds or their vibratory movement during phonation, it is known to apply a stroboscope technique.

It is known to apply in a stroboscope system a flash light with short duration flashes and short recharge time in order to illuminate the cyclic movement object. When the flash bulb is periodically triggered with the same or a slightly different frequency compared to the vibratory frequency of the vibratory object, the object, which vibrates with a high speed, will be observed as immobile or as moving with a slow motion pattern. However, a disadvantage of this system is that the maximum flash frequency of an ordinary bulb/light source-stroboscope is limited and typically does not exceed 1 000 flashes per second maximally, with a flash duration of about 10 to 30 microseconds. Furthermore, to obtain a stroboscopic image with a sufficiently high quality, it has to be dark, at least around the observed object.

Moreover, the traditional flash stroboscope system typically has two kinds of light sources, a normal light source for navigating the imaging system to a proper position, and the other light source is the flash light for generating flashes. Therefore, the two light sources have different radiation spectra which results in inconsistent hue of the images under the two kinds of illumination.

Generally, the flash light source is a high pressure discharge light source which is operated by an energy transferring circuit, as for example disclosed in US Pat. No. 4,194,143 to Farkas et al. A disadvantage of this energy transferring circuit is that it generates a noise in the frequency range audible by the human auditory system, which not only may be perceived as annoying but may also disturb measurements of e.g. the sounds produced by the human vocal system.

*International patent publication* WO 00/33727 to Hess et al. *and United States patent No* US6, 734, 893 to Hess et al disclose a stroboscopic system in which a, high power, light-emitting diode (LED) is applied as a flash light source. The stroboscopic system has an illumination system, with four light emitting diodes placed at the tip of a rigid endoscope. The LEDs are controlled by an electric control unit to generate pulses of light. The pulses of light illuminate moving parts of the human body, and enable an observer to obtain information about the dynamical behaviour of intracorporal structures, e.g. vocal fold oscillations. However, a disadvantage of the system known from these 'Hess' documents is that a high brightness of the LEDs is required, which causes a high degree of heat dissipation, which can cause burning of the cavity of the human body in which the rigid endoscope is inserted, e.g. the mouth. The heat dissipation also reduces the life span of the LEDs.

It is also known in the art of stroboscopy, to use a continuous light source and to interrupt the illumination of the object by positioning a rotating disc provided with holes between the light source and the object to be illuminated, which is as the Oertel principle. However, this apparently simple system is cumbersome and rarely applied in practice because it has to be triggered by the person being examined and the examiner. More in particular, the examinee has to provide a voice signal which corresponds to the stroboscope frequency controlled by the examiner.

An alternative is known in which the rotating disc is present in the light path between the object and a camera. Accordingly, a static image or a slow motion of the object will be registered by the camera, although an observer cannot perceive the phenomenon directly but has to view the image or sequence of images registered by the camera. However, this alternative has the same drawbacks because of the mechanical rotating disc. Furthermore, a mechanical noise will be introduced.

Following the above-mentioned alternative, an electronic shutter of a solid state imaging device, such as Charge Coupled Device (CCD) or Complementary Metal-Oxide-Semiconductor (CMOS) imaging device, brings a new strategy in stroboscopy by using continuous lighting. The electronic shutter can be switched between on and off by external electronic pulses which are related to the vibratory frequency of the object observed.

Regardless of the manner in which the stroboscopic effect is obtained, e.g. by interrupting the projection of light onto the object or periodically inhibiting the reception of light transmitted or reflected by the object, in a stroboscope system, the operating frequency, i.e. the frequency with which the lights source has to flash or, the disk has to rotate, or the electronic shutter is to be switched on or off, has to be controlled. That is, the operating frequency has to be set to a value suitable to perceive the observed object as motionless or moving slowly. For some applications, the frequency may be known in advance, e.g. in case the object has a constant vibration frequency which can be determined a priori. The operating frequency can in such case be set to a *fixed value before the stroboscope system is switched on.

However, for other applications a triggering system may be required which determines the vibration frequency, e.g. in case the vibration frequency varies in time. For example, the 'Hess' documents referred to above, disclose an Fo-detector which measures the fundamental frequency of the sound generated by the vocal folds and determines from the measured frequency a suitable operating frequency.

It is further known to use a digital signal processing (DSP) technique to determine the fundamental frequency of the voice signal from a microphone. However, a disadvantage is that it is very difficult to measure the fundamental frequency accurately, because in chest voice signal the dominating frequency typically is the second harmonic of the fundamental frequency. For instance, it is impossible to obtain a fundamental frequency from a non-periodic signal.

In the art of laryngostroboscopy, a triggering system has a contact vibration sensor, also referred to as a contact microphone, to determine the vibration frequency of vocal folds. A contact vibration sensor is a sensor which is placed on the skin of an individual to be observed, and which measures the vibration of the skin. Compared to a regular microphone, which basically measures the frequency of oscillations in the air, a contact vibration sensor placed on the neck near the larynx measures a signal in which the fundamental frequency of the vibration of the vocal folds dominates. The fundamental frequency can therefore be determined more accurately and in a less complex manner compared to a regular microphone. Accordingly, the operating frequency will, compared to the microphone based determination, correspond more accurately to the vibration frequency of the vocal folds. However, a disadvantage is that it is difficult to get vibratory information when the sensor is not placed in the correct position or the sensor moves out of the correct position. Accordingly, the contact vibration sensor has be fixated to the subject's neck and be placed accurately, near the larynx. Moreover, the contact pressure of the contact vibration sensors will influence the original phonation status of the larynx, and hence will influence medical examination.

It is also known to determine the vibrating frequency by measuring the impedance changes due to a low current flow across the neck in the vicinity of the vocal folds, using the signal from electroglottography (EGG), the most widely used glottographic technique. From the impedance changes, the vibration frequency can be determined as well. However, like the contact vibration sensor, this requires an accurate positioning of the probing electrodes on the skin, near the larynx and mignt influence the medical examination.

Accordingly, a common disadvantage of the prior art triggering mechanisms described above is that it is difficult to obtain accurate frequency information from the vibratory object.

### SUMMERY OF THE INVENTION

It is an object of the invention to provide a triggering system for stroboscopy which is able to obtain accurate frequency information of a vibratory object. Therefore, according to the invention, a triggering system according to claim 1 is provided.

Such a triggering system allows more accurate frequency information to be obtained because the vibratory information is obtained from the light reflected or transmitted from vocal folds in response to the projected light. Hence, the vibratory information is obtained from the vocal folds in a more direct manner, and is less likely to be influenced by other objects. Accordingly, the vibratory information is more accurate.

An additional advantage which may be obtained with such a triggering system is that, since the vibratory information is converted into triggering signals for the stroboscope system, the stroboscope system will allow an improved observation of the vocal folds.

Another additional advantage which may be obtained with such a triggering system, is that the vibratory information can be obtained with less computational effort, for example by determining the interval between maxima in the light received from or transmitted by the vocal folds.

Another additional advantage which may be obtained with such a triggering system is that it can be implemented in the stroboscopic system and be applied substantially without additional invasive procedure or contact between the stroboscopic system and the vocal folds or the surrounding environment of the vocal folds.

Further, according to the invention, a method according to claim 11 is provided.

According to a preferred embodiment of the invention, a stroboscope system according to claim 12 is provided.

According to another preferred embodiment of the invention, a method for performing stroboscopy according to claim 13 is provided.

Other preferred embodiments of the invention are set forth in the dependent claims.

### BRIEF DESCRIPTION OF THE DRAWINGS

Further details, aspects and embodiments of the invention will be described, by way of example only, with reference to the drawings.
FIG. 1 shows a schematic view of an example of a preferred embodiment of an optical triggering system of a stroboscope applied to observe vocal folds.
FIG. 2 shows an example of a spectral response curve of a beam splitter to separate visible and near infrared light.
FIG. 3 shows an example of a spectral response curve of a beam splitter to separate the visible light by different proportions.
FIG. 4 shows a schematic block diagram of an example of an implementation of a driving circuit and fundamental frequency detector suitable for use in the example of FIG. 1.
FIG. 5 shows a schematic block diagram of another example of an implementation of a driving circuit and fundamental frequency detector.
FIG. 6 shows a vibratory image of normal vocal folds from line-scan imaging device.
FIG. 7 shows a vibratory image of abnormal vocal folds from line-scan imaging device.
FIG. 8 illustrates an application of the presented invention.
FIG. 9 schematically shows a further example of an embodiment of an imaging system.

### DETAILED DESCRIPTION

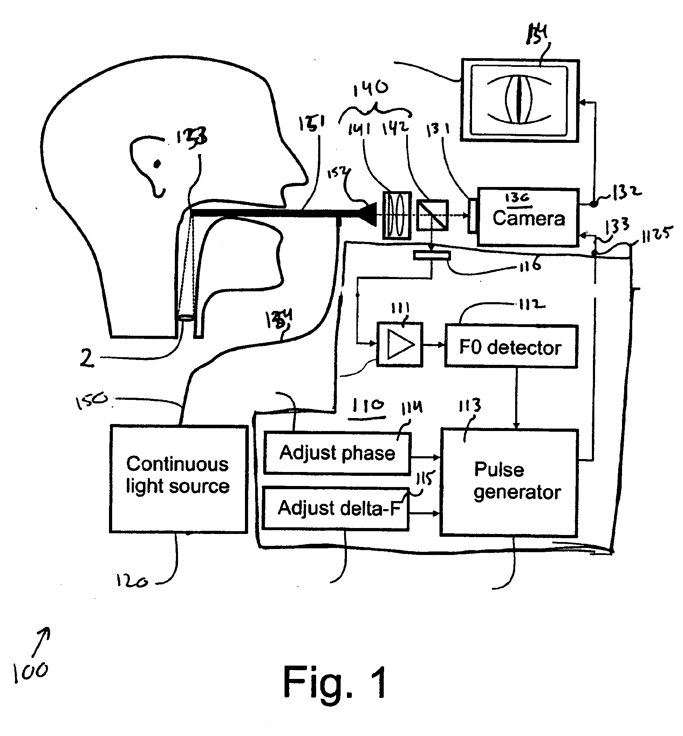

Referring first to FIG. 1, an example of an embodiment of a triggering system **110** is shown, implemented in a stroboscopic system **100.** By way of example, FIG. 1 illustrates the application of the stroboscopic system in medical stroboscopy, more in particular in laryngostroboscopy. In this respect, *laryngoscopy* refers to an examination of the larynx (voice box). A *laryngoscope* refers to an instrument that is used to obtain a view of the larynx. *Glottography* is a general term used for methods to monitor the vibrations of the glottis, i.e. the vocal folds, which is a part of the larynx. However, the invention is not limited to medical stroboscopy, but can also be applied in industrial stroboscopy.

The shown example of a stroboscopic system **100** includes a triggering system **110,** a light source **120** which in an active state projects light onto an object **2** and an image generating system 130 which in an active state generates an image of the object 2 from light reflected from and/or transmitted by the object **2.** Of course, when the image generating system 130 is in the active state and the object **2** does not reflect or transmit light, the image generating system 130 is not able to generate the image.

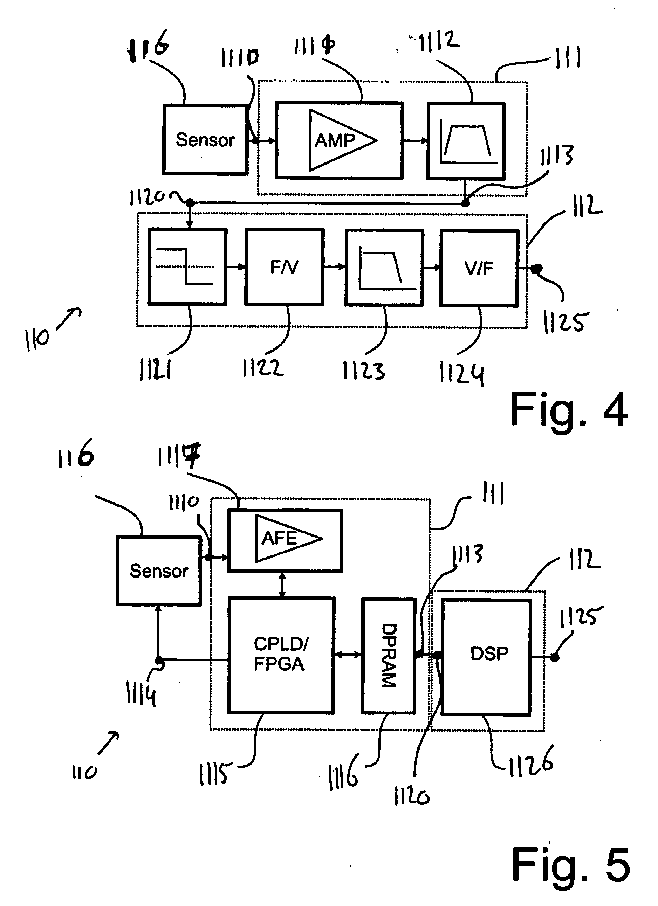

In the example of FIG. 1, the triggering system **110** includes an optical sensor **116** which can sense light reflected or transmitted from the vibratory object **2** in response to the projected light, in order to obtain vibratory information from the vibratory object 2. The triggering system **110** further includes an electronic control arrangement **111-115** connected to the optical sensor, which can convert the vibratory information into triggering signals for the stroboscope system **100.** The electronic control arrangement **111-115** may for example be implemented as is described below in more detail with reference to FIGs. 4 and 5.

As shown in FIG. 1, the electronic control arrangement **111-115** includes a control output **1125** for outputting the triggering signals to the stroboscope system. The control output **1125** is connectable to other parts of the stroboscope system. More in particular, in FIG. 1, the control output **1125** is connected to an imaging control input **133** of the image generating system 130. The triggering signals provide the part of the stroboscopic system **100** connected to the control output **1125** with information about a determined vibration frequency of the vibratory object **2.** Accordingly, in the example of FIG. 1, the imaging device can be operated with an operating frequency which is substantially the same as the determined vibration frequency to observe the vibratory object **2** as motionless or with an operating frequency slightly different from the determined vibration frequency to observe the object **2** as moving slowly.

In the example of FIG. 1, the image generating system 130 is switched between the active state and a non-active state in accordance with the triggering signals while the light source **120** remains active, i.e. the light source **120** is a continuous source. As shown in FIG. 1, the image generating system 130 is optically connected to the triggering system **110,** for receiving triggering signals from the triggering system **110.** However, depending on the specific implementation, the light source may be switched between the active state and a non-active state in accordance with the triggering signals while the image generating system 130 remains, substantially without interruptions, active. Also, both the image generating system 130 and the light source **120** may be switched between the active and the non-active state in accordance with the triggering signals.

As shown in FIG. 1, the image generating system 130 has an optical element **131** at which the light from an object can be received and converted into an image. For example the optical element **131** may include a two-dimensional matrix-shaped arrangement of opto-electrical converters, which convert light incident into an electric signal. The electric signals from the arrangement of opto-electrical converters may then be processed, for example to be converted into a video stream or otherwise, and be outputted at an imaging output **132** to a display **134** at which the image is outputted in a manner suitable to be perceived by the naked eye.

In the example of FIG. 1, the stroboscopic system **100** further includes an optical system **140** which projects the light transmitted from or reflected by the object **2** onto the optical sensor **116** and/or the image generating system 130. The light source **120** is optically connected to a projection system **150** which provides a light path from the source **120** to the object **2** and projects the light generated by the source **120** onto the object **2.** However, depending on the specific implementation the light generated by the lights source **120** may be projected directly onto the object **2.**

In this respect, it should be noted that in this application the term *'light'* refers to electro-magnetic radiation with a frequency in the range from infrared to ultraviolet. The term' *visible light'* refers to electro-magnetic radiation a frequency in the range which can be perceived by the naked human eye whereas the term *'invisible light'* refers to electro-magnetic radiation a frequency in the range which the naked human eye is not able to perceive and e.g. includes far infrared and ultraviolet radiation.

The stroboscopic system may observe one or more objects of any suitable type. The object **2** may for example include a part of a body of an animal, such as a human body. In FIG. 1, for instance, the observed vibratory objects are the vocal folds of a human. In phonation, the vibratory frequency of vocal folds ranges from about 70 Hz to 1 kHz, depending inter alia on gender, age, phonetic pitch. Vibrations at these frequencies are too fast to be observed by the naked human eye. Accordingly, a stroboscopic system allows an observation of the vocal folds, and therefore enable a medical examination thereof. However, the object **2** may also be of a different type.

The light generated by the source **120** may be projected onto the vibratory object **2** in any manner suitable for the specific implementation, for example directly, or via a flexible optical fibre, or optical elements such as mirrors and/or lenses. In the example of FIG. 1, for instance, the light source **120** is optically connected to a projection system **150** which can project light onto a vibratory object **2** which cannot be observed directly. More in particular, the light is projected via an endoscope **151.** The endoscope **151** can be used for diagnostic purposes, and in particular to observe an inside part of the human body. The endoscope **151** in this example is dimensioned such that it forms an indirect laryngoscope. That is, because of the location of the larynx behind the tongue, a 90 or 70 degree endoscope is needed. The endoscope **151** can hence be used, as illustrated in FIG. 1, to visually observe the larynx, and in particular to observe the glottis.

In this example, the light generated by the source **120** is guided by an optical wave guide, in this example a flexible optical fibre **154,** to the endoscope **151** which can be inserted partially into the mouth of the human, such that light projecting out of the endoscope **151** at a terminal end **153** thereof projects into the throat and is incident on the vocal folds. This enables an indirect observation of the vocal folds, which cannot be observed directly because they are located in the throat.

As shown in FIG. 1, the endoscope **151** may include a tubular element which provides a light path from the optical wave guide **154** to a terminal end **153** of the tubular element. At the terminal end **153,** light from the source **120** fed into the tubular element by means of the fibre **154** is projected out of the tubular element onto the vibratory object **2.** In FIG., 1, in operating position, i.e. in such a position that the larynx is illuminated, the longitudinal axis of the tubular element extends more or less from the entrance of the mouth to the throat and at the terminal end **153,** the light from the light source **120** (provided via the optical fibre **154**) is projected at a non-parallel angle with respect to the longitudinal axis of the tubular element, for example between 70° and 90°.

The endoscope **151** may have three paths (not shown in the figure), an imaging optical path, a light transmission path, and an air flow path. The light from the source **120** propagates along the light transmission path, which extends from the entrance point of the optical fibre **154** to the terminal end **153.** The light reflected from the object **2** propagates along the imaging optical path, which extends from the terminal end **153** to a second end **152** at a distance from the first end **153.** To improve the efficiency of the light transmission, that is, to reduce the loss of light on the interface between the optical fibre **154** and the endoscope **151,** an endoscope with an integrated optical fibre may be used.

In the example of FIG. 1, the endoscope **151** is optically connected, at a second end **152** at a distance from the first end **153,** to the optical system **140,** i.e. at the second end **152,** the light is projected onto the optical system **140.**

The optical system **140** may be implemented in any manner suitable to project the light from the object **2** onto the image generating system 130 and/or the optical sensor **116,** and may for example include an arrangement of lenses and other optical elements which focuses the light on the (surface of) the image generating system 130 and/or the optical sensor **116** and/or separates the light into at least two different beams.

In the example of FIG. 1, the optical system **140** for instance includes an optical adaptor **141.** The optical adaptor **141** can focus the image on the image generating system 130 and/or on the optical sensor **116.**

Between the optical adaptor **141** and the image generating system 130, a beam splitter **142** is present. The beam splitter **142** divides the beam of light from the object, e.g. in FIG. 1 outputted at the second end **152** of the endoscope **151,** into two sub-beams, as indicated with the arrows in FIG. 1. A first beam is directed via a first optical path to the sensor(s) of the video camera **130** and a second beam is directed via a second optical path to an optical sensor **116.** The optical sensor **116** may for example be a single photodiode, a line imaging device, such as a linear CCD sensor, or a matrix imaging device or any other type of optical sensor suitable for the specific implementation.

The first beam and the second beam may have a different frequency spectrum. For example, the beam splitter may split the incident light in a first beam with substantially only visible light and a second beam with substantially only invisible light. Thereby, only components in the light transmitted from or reflected by the object **2** are transmitted to the optical sensor **116** to which the image generating system 130 is not sensitive, and hence which are not relevant for the image quality. Hence, the quality of the image generated by the image generating system 130 is (almost) not influenced by the triggering system, since most of the visible light is projected via the beam splitter onto the image generating system 130. However, it is also possible that the optical sensor **116** and the image generating system 130 receive light with substantially the same frequency spectrum, for example in case the triggering **116** is based on information derived in the same frequency range as the image generating system 130, e.g. visible light.

The light incident on the optical sensor is converted into signals suitable to be processed by the circuitry in the triggering system **110.** The triggering system may for instance include a pre-processing unit **111** connected to an output of the sensor **116.** The pre-processing unit **111** pre-processes the signal to be suitable to be received by a frequency determining unit **112,** which in this example determines the fundamental frequency of the movement of the observed object. The output signal of the frequency determining unit is transmitted to a pulse generator **113.** The output signal may for example be a square wave signal with the same frequency as the fundamental frequency.

The pulse generator **113** generates triggering pulses for the image generating system 130, which may for example be transmitted to a triggerable electronic shutter of image generating system 130. The triggering system **110** has controls by means of which the triggering system can be adjusted, for example manually. The triggering system **110** includes a phase adjustment control **114** and a delta-F adjustment control **115.**

In the motionless mode, the triggering frequency is the same as the fundamental frequency of the vocal folds, and a 'frozen'-like image is shown on a display **134.** By means of the phase adjustment control **114** the start triggering position may be adjusted to reveal a still-standing image but with a different phase of the cycle of movement of the object.

By means of a delta-F adjustment control **115,** the mode of the stroboscopic system can be controlled. The value inputted by means of the delta-F adjustment control **115** is added to the frequency determined by the unit **112.** Hence, in the example the frequency of output triggering pulses is the sum of the frequency of vocal folds and the delta-F. When delta-F is zero, the output frequency is equal to the frequency of vocal folds, and therefore a still image (in FIG. 1 of the vocal folds) is obtained. When delta-F is set to a value not equal to zero, a (slow) movement of the object will be perceived. While changing the delta-F, the frequency of output triggering pulses for camera changes relative to the fundamental frequency of vocal folds, which results in the (slow) motion changing correspondingly.

The triggering system **110** may be implemented as shown in figs. 4 or 5, in which for sake of simplicity the triggering unit **113** and the controls **114,115** are omitted. However, the triggering system **110** may also be implemented in another manner suitable to generate from the received light triggering signals representing vibration information about the object.

In the example of FIG. 4, for instance the optical sensor **116** is connected to an electronic control arrangement **111-115.** The electronic control arrangement includes a pre-processing unit **111** which is connected with a pre-processor input **1110** to the output of the optical sensor. A frequency determining unit **112** is connected with an determining input **1120** to a pre-processor output **1113.** The pre-processing unit **111** can process the signal received from the optical sensor **116** such that it is suitable for the frequency determining unit **112.**

In this example of FIG. 4, the optical sensor **116** outputs a signal which represents the intensity of the light at a certain moment. E.g. the optical sensor **116** may for example be an opto-electrical converter, such as a photodiode, which outputs a current or a voltage proportional to the intensity of the incident light. Thereby, the optical sensor may be of a simple design. Since the reflective light fluctuates with the vibration of the vocal folds, the fluctuation in current or voltage represents the vibration of the object **2**.

In the example of FIG. 4, the pre-processing unit **111** includes an amplifier **1111** and a band pass filter **1112.** The output of the optical sensor, e.g. the photodiode, is amplified by the amplifier **1111.** The band pass filter **1112** is used to allow only passing of the fundamental frequency, that is, it not only removes the low frequency component influences, such as the fluctuation of light source and displacement of observing position, but also eliminates high frequency noise. A suitable pass band of the band pass filter **1112** for applications in laryngoscopy is found to be a 3 dB cut-off frequency between 50 Hz and 1 kHz.

The output from the band pass filter **1112** is sent to the frequency determining unit **112.** Because the overall intensity of the light from the vocal folds is directly related to the fundamental frequency of the vocal folds without the influences of vocal tract, the unit **112** shown in the example of FIG. 4 can determine the vibration information accurately from the output of the optical sensor 116. The frequency determining unit **112** includes a comparator **1121** connected to a determining input **1120.** The comparator **1121** acts as a zero crossing detector and generates a square wave output at the applied frequency. That is, the comparator **1121** outputs a constant signal at a first level in case the output of the pre-processing unit is above a threshold and a constant signal at a second level in case the output of the pre-processing unit is below a threshold. The first level may for example be a positive voltage and the second level a negative voltage and the threshold may be set to zero. However, in case e.g. the output of the pre-processing unit **111** has an off-set, the threshold may be set to correspond to the off set.

The output of the comparator **1121** is fed into a frequency-to-voltage converter **1122** that produces an output of which the amplitude proportional to the frequency of the signal inputted to the converter **1122.** The output is smoothed by a low pass filter **1123** to avoid frequency jumps and then a voltage-to-frequency converter **1124** is used to convert the smoothed signal to a square wave with a 50% duty cycle, of which the frequency is proportional to the input voltage. The square wave may, as e.g. shown in FIG. 1 be outputted at the trigger output **1125** in order to alternately switch the image generating system **130** between an active state and a non-active state.

The optical sensor **116** may be sensitive to light in any suitable frequency band. For example, the optical sensor **116** may be sensitive to invisible light, such as infrared or ultraviolet light. Thereby, the optical sensor 116 can accurately detect light while the interference with the imaging system is reduced. Especially in case the optical sensor **116** is sensitive to near infrared NIR radiation, the light reflected by the object **2** contains enough NIR components to be responded by a sensor, even if in the light path, e.g. between the light source and the object, an infrared cutoff filter is present (e.g. to reduce the amount of heat transmitted through the optical system).

To keep colour consistency of the image from image generating system 130 with what is perceived by the human naked eye, the image generating system 130 may have a spectral response similar to that of the human eyes.

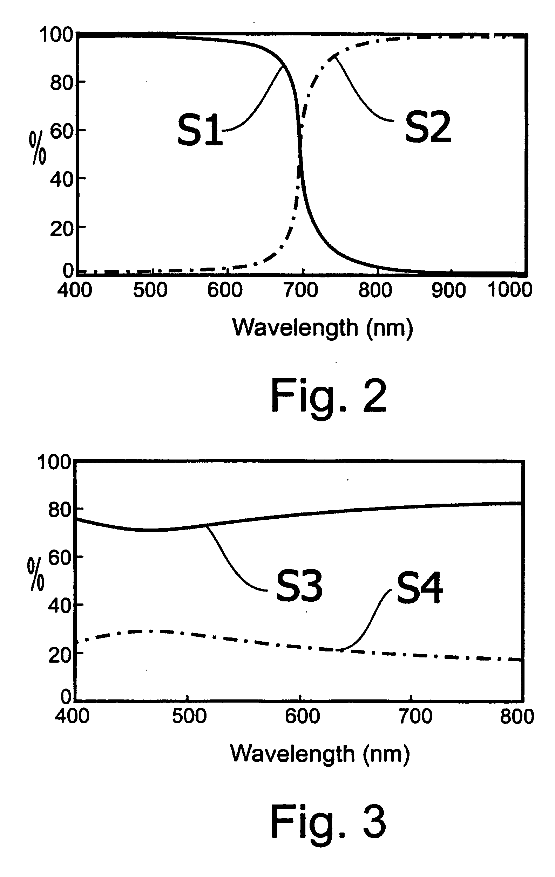

As mentioned, the light from the object **2** may be spitted in a first beam, incident on the image generating system 130, and a second beam, incident on the optical sensor **116,** with different spectra. FIG. 2 shows an example of a spectral response curve suitable for an imaging device sensitive to visible light and for an optical detector sensitive to near infrared radiation. For instance in the example of FIG. 1, the beam splitter **142** may divide the light incident thereon, containing both visible and NIR components, in two ways, one way is transmission with spectral response curve **S1,** and the other way is reflection with spectral response curve **S2.** As shown in FIG. 2, the first beam may for example consist only of light with a wavelength below about 0.7 micrometer, whereas the second beam may for example consist only of light with a wavelength above about 0.7 micrometer. The transmitted light may then be projected onto the imaging optics **131** of the image generating system **130** and the reflected light on the optical sensor **116.** Thereby, only power outside the band to which the image generating system 130 is sensitive is diverted to the optical sensor **116** and the image quality is improved compared to a beam splitter which splits the light into beams with similar spectra.

The detection circuit may be arranged to determine from the vibratory information a vibratory frequency of two or more different parts of the vibratory object, such as the vibratory frequency of different vocal folds in the voice box of a human being. In normal vocal folds, the vibrations of the two sides are periodical and symmetric. The fundamental frequencies from each of the two vocal folds are exactly or approximately the same. Therefore, which vocal fold is used to determine the fundamental frequency does not affect the triggering.

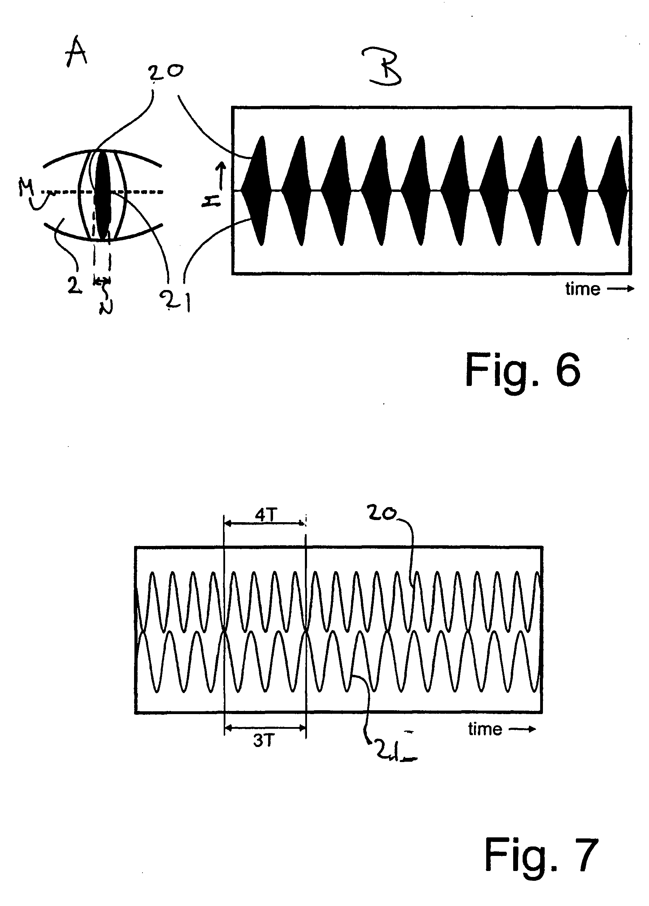

However, in same pathological cases, such as unilateral laryngeal paralysis, it is very difficult to find a frequency suitable for triggering using the prior art techniques, e.g. from the voice or contact vibratory sensor, because one left and right vocal fold are vibrating differently. The example FIG. 7 shows a typical vibratory wave of left laryngeal paralysis derived from a linear scan image or videokymogram, as is explained below, which reveals that the vocal fold at the left side 20 vibrates at a higher frequency than the right one 21, in FIG. 7 the period ratio between left and right vocal fold is 4:3. For example, if the fundamental frequency of the left vocal fold is 200Hz and the frequency of the right vocal fold is 150Hz, the voice from the patient is diplophonic and bitonal hoarse. Therefore, for a traditional stroboscope system, it is impossible to obtain a stable slow motion vibration image of vocal folds, which is the main complaint point about stroboscopy from physicians and phoniatricians.

However, in case the determining circuit **112** is able to determine the fundamental frequency (or other vibratory information) of each of two or more vibratory objects, by selecting a determined frequency to generate the triggering signals from, the object corresponding to this frequency can be perceived as motionless or moving slowly and may hence be observed, and in case observation of another object is desired, the fundamental frequency of the other object can be used to generate the triggering signal. E.g. in case the object is the glottis, in case observation of the vibration of a right vocal fold is desired, the fundamental frequency of the right vocal fold is used to trigger the stroboscope and a slow motion of the right vocal fold can be observed, while the left vocal fold will be observed as blurred, because of the frequency difference. Likewise, in case observation of the vibration of the left side vocal fold is desired, the fundamental frequency of the left vocal fold is used to trigger the stroboscope and a slow motion image of the left vocal fold can be obtained while the image of the right vocal fold will be blurred.

In the example of FIG. 5, for instance, the optical sensor **116** may be a linear sensor, which can obtain a line-shaped vibratory image, named videokymogram. The linear sensor may for example be implemented as is described in J. Svec, H.K.Schutte, "Videokymography: high-speed line scanning of vocal fold vibration". J. Voice, 1996; 10: 201-205, relevant parts incorporated herein by reference. Using a linear imaging device, it is much easier to obtain accurate vibratory information, e.g. as a high resolution vibratory image of the vocal fold, than using an imaging system which generates a two-dimensional system. Compared to a single photodiode system, a linear scan sensor system allows more information to be obtained, such as in case the object is a vocal fold, the vibration, the collision and the mucosal waves of both vocal folds (left and right vocal fold), which can be shown synchronously.

FIG. 6 schematically illustrates the operation of a linear sensor. As shown in FIG. 6A, the linear sensor generates a line-shaped image of the object, e.g. in the example the vocal folds, along the line denoted with M in FIG. 6, which may for example be a 1-by-M pixel image. FIG. 6B illustrates the image M as a function of time. In FIG. 6B, the black part corresponds to the opening between the left and right vocal folds, i.e. as indicated in FIG. 6A with arrow N. The black part in FIG. 6B hence represents the vibration of the glottis **2** along the selected line, while the edges of the black part represent the vibration of the left vocal fold 20 and the right vocal fold **21,** respectively. Due to the vibratory movement of the vocal folds **2,** the position of the edges changes in time. By determining the period between the peaks in an edge, the fundamental frequency of the vibration of the corresponding vocal fold **20,21** can be determined. Accordingly, the vibration of the vocal folds and differences between the vibrations of the left and right vocal fold **20,21** can be determined.

When the optical sensor **116** is a linear sensor, the sensor **116** may be sensitive to visible light. In case the example of FIG. 5 is implemented in the example of FIG. 1, to obtain a high SNR (signal to noise ratio) stroboscopic image, it is found to be suitable to divert more than 70% of the visible light to the image generating system 130, and about 30% or less of the visible light to the optical sensor **116.** In such case, the spectral response curves of beam splitter **142** may for instance be as shown in FIG.3 to obtain a suitable spectrum in the second beam. The input light is then divided by the beam splitter **142** into two ways, one way is transmission with the spectral response curve **S3** shown in FIG. 3, and the other way is reflection with spectral response curve **S4** shown in FIG. 3.

The linear sensor may for instance be connected to a pre-processing unit 111 as shown in fig. 5. The pre-processing unit **111** digitises the analog image signal generated by the linear sensor, and may perform other function such as amplifying. In the example of fig. 5, the pre-processing unit is connected with a pre-processor input **1110** to the optical sensor **116.** The pre-processing unit 111 includes an analog-to-digital converter which converts the signal outputted by the optical sensor **116** into a digital signal. In addition, the signal may be amplified. In the example of fig. 5, the signal is converted and amplified by an analog front end (AFE) **1117.** The conversion speed and resolution may have any value suitable for the specific sensor and the desired image quality. For example, when the linear sensor contains 512 pixels and has a sampling speed of 8000 lines per second, a converter with 5MHz sampling speed and 8-bits gray depth may be used.

The digital signal generated by the AFE **1117** is transmitted to the frequency determining unit **112,** in this example via a complicated programmable logic device (CPLD) or a field programmable gate array device (FPGA) **1115** and a dual port random access memory (DPRAM), which contains two ports, one port connected with the programmable device **1115** and the other port connected with the frequency determining unit DSP platform **1126.** The CPLD/FPGA device **1115** is further connected to a control input **1114** of the sensor **116** and generated a clock signal. The FPGA generates a clock signal which is used as a time base for the operation of the CCD. The FPGA further generates logical signals suitable to transfer the image data from the AFE to DSP **1126.**

In the example of fig. 5, the frequency determining unit **112** includes a digital signal processor (DSP) **1126.** The DSP **1126** analyzes the vibratory image and obtains the two fundamental frequencies of the two vocal folds. The output from DSP is a square wave with a frequency corresponding to the determined fundamental frequency of a selected vocal fold.

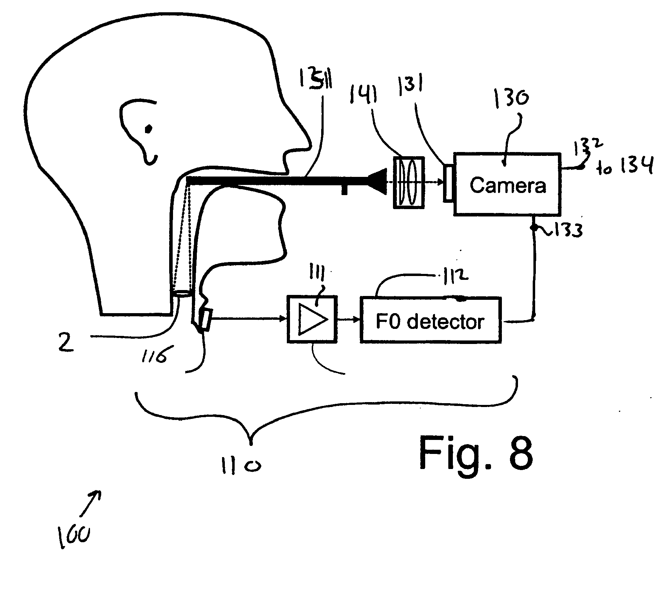

FIG. 8 shows another example of a stroboscopic system **100.** For sake of conciseness, the elements shown in fig. 1 are not described in further detail. In fig. 8, the optical sensor **116** is placed on the skin at the anterior of the neck, about a position corresponding to the subglottal space in the throat. The modulations imposed on the light beam by the vibratory openings and closing of the glottis are transformed to suitable signals by the optical sensor **116,** e.g. into variable voltages by a photodiode or other suitable signals. Accordingly, the optical sensor **116** receives light transmitted or reflected by the object, e.g. the vocal folds in this example, which propagates along a completely different path than the path along which the light received by the image generating system 130 propagates.

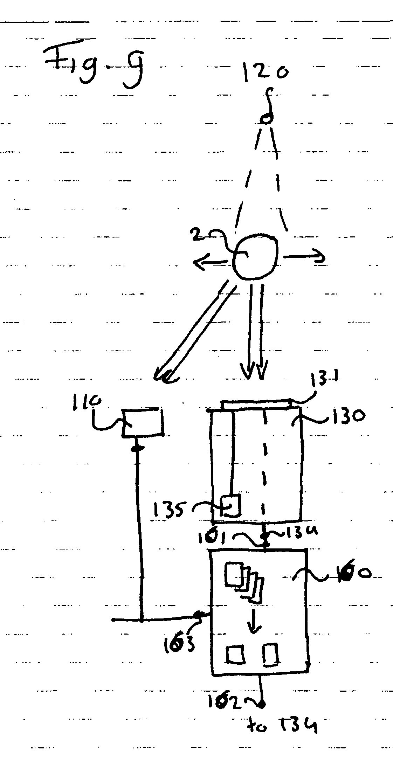

In the examples of figs. 1 and 8, the image generating system **130** is switched between an active state and an inactive state according to the triggering signals. However, as shown in FIG. 9, it is also possible to provide an image processing system **160** between the image generating system **130** and the display **134.** The image processing system **160** may receive at an input **161** from the image generating system 130 a series of images generated at times t₁,t₂ .... tₙ and transmit at an output **162** to the display only images from the series which are generated at times corresponding to the triggering signals received at a control input **163** of the image processing system **160.** In this respect, the image generating system **130,** and the image processing system **160** connected thereto may commonly be referred to as the imaging system. Hence, in the examples of FIGs. 1,8 and 9 the imaging system is activated to generate an image of the object from light reflected from and/or transmitted by the object in accordance with triggering signals outputted by the triggering system **110.**

The invention may also be implemented in a computer program for running on a computer system, at least including code portions for performing steps of a method according to the invention when run on a programmable apparatus, such as a computer system or enabling a programmable apparatus to perform functions of a device or system according to the invention. Such a computer program may be provided on a data carrier, such as a CD-rom or diskette, stored with data loadable in a memory of a computer system, the data representing the computer program. The data carrier may further be a data connection, such as a telephone cable or a wireless connection.

In the foregoing specification, the invention has been described with reference to specific examples of embodiments of the invention. It will, however, be evident that various modifications and changes may be made therein without departing from the invention as set forth in the appended claims. For example, the image generating system 130 may be implemented e.g. as a video camera including a (two-dimensional) array of CCDs or other optical sensors.

Also, the invention is not limited to physical devices or units implemented in non-programmable hardware but can also be applied in programmable devices or units able to perform the desired device functions by operating in accordance with suitable program code. Furthermore, the devices may be physically distributed over a number of apparatuses, while functionally operating as a single device. For example, the pre-processing unit 111 may be implemented as a system of connected processors operating to perform the functions of the triggering unit pre-processing unit 111.

Also, devices functionally forming separate devices may be integrated in a single physical device. For example, the pre-processing unit 111, the frequency determining unit 112 and the image processor 160 may be implemented on a single integrated circuit or as a suitably programmed programmable device.

However, other modifications, variations and alternatives are also possible. The specifications and drawings are, accordingly, to be regarded in an illustrative rather than in a restrictive sense.

In the claims, any reference signs placed between parentheses shall not be construed as limiting the claim. The word 'comprising' does not exclude the presence of other elements or steps than those listed in a claim. Furthermore, the words 'a' and 'an' shall not be construed as limited to 'only one', but instead are used to mean 'at least one', and do not exclude a plurality. The mere fact that certain measures are recited in mutually different claims does not indicate that a combination of these measures cannot be used to advantage.

## Claims

1. A triggering system for a stroboscope system, comprising: a light source for projecting light on a vibratory object, **where the vibratory object are the** vocal folds; an optical sensor for sensing light reflected or transmitted from said vibratory object in response to the projected light, and for obtaining vibratory information from the vibratory object; **characterised in** the system further comprising: a control arrangement connected to the optical sensor, for converting the vibratory information into triggering signals for the stroboscope system, said electronic control arrangement including a control output connectable to said stroboscope system, for outputting said triggering signals to said stroboscope system.

2. A triggering system according to claim 1, further including:
a beam splitter positioned in a light path between said vibratory object and said optical sensor, for splitting said light reflected or transmitted from said vibratory object in response to the projected light into a first beam to be received by an image sensor of said stroboscope system and a second beam to be received by the optical sensor.

3. A triggering system according to claim 2, wherein the first beam contains light of a different wavelength range than the second beam.

4. A triggering system according to claim 3, wherein the optical sensor is sensitive to radiation of a range of wavelength not perceivable by humans, and optionally, wherein the first beam consists of light of a wavelength perceivable by humans and/or the second beam consists of light of a wavelength not perceivable by humans.

5. A triggering system according to any one of claims 2-4, wherein the used beam splitter has a fixed transmission and reflection ratio.

6. A triggering system according to any one of the preceding claims, wherein the optical sensor includes a photodiode for converting said light reflected or transmitted from said vibratory object into electrical signals representing the vibratory information of vibratory object.

7. A triggering system according to any one of the preceding claims, wherein the optical sensor includes a line scan imaging device, for sensing light reflected or transmitted from said vibratory object along a line-shaped area of said object and converting said sensed light into signals representing vibratory information of vibratory object.

8. A triggering system according to any one of the preceding claims, wherein said optical sensor further includes a detection circuit for determining from the vibratory information the vibratory frequency of at least a part of the vibratory object.

9. A triggering system according to claim 8, wherein said detection circuit is arranged to determine from the vibratory information a vibratory frequency of at least two different parts of the vibratory object, such as the vibratory frequency of different vocal folds in the voice box of a human being.

10. A triggering system according to claim 9, further including a switch for selecting a selected vibratory frequency and generating triggering signals based on the selected vibratory frequency.

11. A method for generating triggering signals for a stroboscope system, said method including:
projecting light on vocal folds;
obtaining vibratory information from the vocal folds by at least sensing light reflected or transmitted from said vocal folds in response to the projected light;
converting the vibratory information into triggering signals for the stroboscope system, and optionally:
outputting the triggering signals to said stroboscope system.

12. A stroboscope system, including:
a triggering system according to any one of claims 1-10;
a light source which in an active state projects light onto vocal folds,
an imaging system which in an active state generates an image of said vocal folds from light reflected from and/or transmitted by said vocal folds;
said light source and/or said imaging system being connected to said triggering system, for receiving triggering signals from the triggering system and being switched between said active state and a non-active state in accordance with the triggering signals.

13. A method for performing stroboscopy, including
generating triggering signals with a method according to claim 11;
generating an image of said vocal folds from light reflected from and/or transmitted by said vocal folds;
wherein said projecting light and/or said generating an image is performed in accordance with said triggering signals.

14. A laryngoscope, including a stroboscope system according to claim 12.

## Patentansprüche

1. Triggersystem für ein Stroboskopsystem, umfassend:
eine Lichtquelle zum Projizieren von Licht auf ein schwingendes Objekt, wobei das schwingende Objekt die Stimmlippen sind;
einen optischen Sensor um das vom schwingenden Objekt reflektierte oder ausgesendete Licht als Reaktion auf das projizierte Licht zu detektieren und um Informationen über die Schwingungen von dem schwingenden Objekt zu erhalten;
**dadurch gekennzeichnet, dass** das System außerdem umfasst:
eine Kontrolleinheit, welche mit dem optischen Sensor verbunden ist, um die Informationen über die Schwingungen in Triggersignale für das Stroboskopsystem umzuformen,
wobei die elektronische Kontrolleinheit einen Kontrollausgang umfasst, welcher an das Stroboskopsystem anschließbar ist, um die Triggersignale dem Stroboskopsystem zuzuführen.

2. Triggersystem nach Anspruch 1, weiter umfassend:
einen Strahlteiler, welcher in dem Lichtweg zwischen dem besagten schwingenden Objekt und dem besagten optischen Sensor eingebracht ist, um das vom schwingenden Objekt reflektierte oder ausgesendete Licht als Reaktion auf das projizierte Licht in einen ersten Strahl, welcher von einem Bildsensor des besagten Stroboskopsystems empfangen wird und einem zweiten Strahl, welcher von dem optischen Sensor empfangen wird, verteilt.

3. Triggersystem nach Anspruch 2, wobei der erste Strahl Licht eines anderen Wellenlängenbereichs als der zweite Strahl besitzt.

4. Triggersystem nach Anspruch 3, wobei der optische Sensor auf Strahlung eines Wellenlängenbereichs anspricht, der nicht von Menschen wahrnehmbar ist und optional, wobei der erste Strahl aus Licht einer Wellenlänge besteht, der menschlich wahrnehmbar ist und/oder der zweite Strahl aus Licht einer Wellenlänge besteht, die von Menschen nicht wahrnehmbar ist

5. Triggersystem nach einem der Ansprüche 2 bis 4, wobei der benutzte Strahlenverteiler ein festes Verhältnis zwischen Aussendung und Reflektion hat.

6. Triggersystem nach einem der vorhergehenden Ansprüche, wobei der optische Sensor eine Fotodiode umfasst, um das vom schwingenden Objekt reflektierte oder ausgesendete Licht in elektrische Signale umzuwandeln, welche Informationen über die Schwingungen des schwingenden Objekts wiedergeben.

7. Triggersystem nach einem der vorhergehenden Ansprüche, wobei der optische Sensor ein Bilderfassungsgerät nach dem Strichrasterverfahren umfasst, um das vom schwingenden Objekt reflektierte oder ausgesendete Licht entlang eines linienförmigen Bereichs des Objekts zu detektieren und das detektierte Licht in Signale umzuwandeln, welche Informationen über die Schwingungen des schwingenden Objekts wiedergeben

8. Triggersystem nach einem der vorhergehenden Ansprüche, wobei der besagte optische Sensor weiterhin eine Erkennungsschaltung umfasst, um die Schwingfrequenz von mindestens einem Teil des schwingenden Objektes aus den Informationen über die Schwingungen zu ermitteln.

9. Triggersystem nach Anspruch 8, wobei die Erkennungsschaltung so ausgeführt ist, dass aus den Informationen über die Schwingungen eine Schwingfrequenz mindestens zweier unterschiedlicher Teile des schwingenden Objektes ermittelt werden, wie die Schwingfrequenz zweier unterschiedlicher Stimmlippen des Kehlkopfes eines Menschen.

10. Triggersystem nach Anspruch 9, weiterhin umfassend einen Schalter, um eine ausgewählte Schwingfrequenz auszuwählen und Triggersignale aufgrund der ausgewählten Schwingfrequenz zu erzeugen.

11. Verfahren zur Erzeugung von Triggersignalen für ein Stroboskopsystem, umfassend:
projizieren von Licht auf Stimmlippen;
erhalten von Informationen von den Stimmlippen, indem mindestens reflektiertes oder ausgesendetes Licht der Stimmlippen als Reaktion auf das projizierte Licht detektiert wird;
umwandeln der Informationen über die Schwingungen in Triggersignale für das Stroboskopsystem und optional:
weiterleiten der Ansteuersignale an das Stroboskopsystem.

12. Stroboskopsystem, umfassend:
ein Triggersystem nach einem der Ansprüche 1 bis 10;
eine Lichtquelle, welche in eingeschaltetem Zustand Licht auf Stimmlippen projiziert;
ein Bilderfassungssystem, welches in eingeschaltetem Zustand ein Bild der Stimmlippen aus dem reflektierten und/oder ausgesendeten Licht der Stimmlippen erzeugt;
die Lichtquelle und/oder das Bilderfassungssystem ist mit dem besagten Triggersystem verbunden, um Triggersignale von dem Triggersystem zu erhalten und zwischen dem eingeschalteten Zustand und einem ausgeschalteten Zustand abhängig vom Triggersignal geschaltet zu werden.

13. Verfahren zur Ausführung von Stroboskopie, umfassend:
erzeugung von Triggersignalen durch ein Verfahren nach Anspruch 11;
erzeugung eines Bildes der Stimmlippen aus von den Stimmlippen reflektiertem und/oder ausgesendetem Licht,
wobei das Projizieren des Lichts und/oder die Erzeugung eines Bildes in Abhängigkeit der Triggersignale durchgeführt wird.

14. Laryngoskop, umfassend ein Stroboskopsystem nach Anspruch 12.

## Revendications

1. Système de déclenchement pour un système stroboscopique, comprenant :
une source de lumière destinée à projeter de la lumière sur un objet vibrant, l'objet vibrant étant des cordes vocales ;
un capteur optique destiné à capter de la lumière réfléchie ou transmise provenant dudit objet vibrant en réponse à la lumière projetée, et à obtenir de l'information vibratoire de l'objet vibrant ;
**caractérisé en ce que** le système comprend en outre :
un agencement de commande raccordé au capteur optique, destiné à convertir l'information vibratoire en signaux de déclenchement pour le système stroboscopique, ledit agencement de commande électronique incluant une sortie de commande pouvant se connecter audit système stroboscopique, pour sortir lesdits signaux de déclenchement vers ledit système stroboscopique.

2. Système de déclenchement selon la revendication 1, incluant en outre un diviseur de faisceau placé dans un trajet de lumière entre ledit objet vibrant et ledit capteur optique, destiné à diviser ladite lumière réfléchie ou transmise, provenant dudit objet vibrant en réponse à la lumière projetée, en un premier faisceau destiné à être reçu par un capteur d'image dudit système stroboscopique et un second faisceau destiné à être reçu par le capteur optique.

3. Système de déclenchement selon la revendication 2, dans lequel le premier faisceau contient de la lumière d'une gamme de longueurs d'onde différente du second faisceau.

4. Système de déclenchement selon la revendication 3, dans lequel le capteur optique est sensible au rayonnement d'une gamme de longueurs d'onde non perceptible par l'homme, et facultativement, dans lequel le premier faisceau est constitué de lumière d'une longueur d'onde perceptible par l'homme et/ou le second faisceau est constitué de lumière d'une longueur d'onde non perceptible par l'homme.

5. Système de déclenchement selon l'une quelconque des revendications 2 à 4, dans lequel le diviseur de faisceau utilisé a un rapport fixe de transmission et réflexion.

6. Système de déclenchement selon l'une quelconque des revendications précédentes, dans lequel le capteur optique inclut une photodiode destinée à convertir ladite lumière réfléchie ou transmise provenant dudit objet vibrant en signaux électriques représentant l'information vibratoire de l'objet vibrant.

7. Système de déclenchement selon l'une quelconque des revendications précédentes, dans lequel le capteur optique inclut un dispositif d'imagerie à balayage ligne par ligne, destiné à capter la lumière réfléchie ou transmise provenant dudit objet vibrant le long d'une zone en forme de ligne dudit objet et à convertir ladite lumière captée en signaux représentant l'information vibratoire de l'objet vibrant.

8. Système de déclenchement selon l'une quelconque des revendications précédentes, dans lequel ledit capteur optique inclut en outre un circuit de détection destiné à déterminer à partir de l'information vibratoire la fréquence vibratoire d'au moins une partie de l'objet vibrant.

9. Système de déclenchement selon la revendication 8, dans lequel ledit circuit de détection est agencé pour déterminer à partir de l'information vibratoire la fréquence vibratoire d'au moins deux parties différentes de l'objet vibrant, comme la fréquence vibratoire de différentes cordes vocales dans la cavité vocale d'un être humain.

10. Système de déclenchement selon la revendication 9, incluant en outre un commutateur destiné à choisir une fréquence vibratoire choisie et à engendrer des signaux de déclenchement en se basant sur la fréquence vibratoire choisie.

11. Procédé de génération de signaux de déclenchement pour un système stroboscopique, ledit procédé incluant ;
la projection de lumière sur des cordes vocales ;
l'obtention d'une information vibratoire des cordes vocales au moins en détectant de la lumière réfléchie ou transmise provenant desdites cordes vocales en réponse à la lumière projetée ;
la conversion de l'information vibratoire en signaux de déclenchement pour le système stroboscopique, et facultativement :
la sortie des signaux de déclenchement vers ledit système stroboscopique.

12. Système stroboscopique, incluant :
un système de déclenchement selon l'une quelconque des revendications 1 à 10 ;
une source de lumière qui, à l'état actif, projette de la lumière sur des cordes vocales ;
un système d'imagerie qui, à l'état actif, engendre une image desdites cordes vocales à partir de la lumière réfléchie et/ou transmise par lesdites cordes vocales,
ladite source de lumière et/ou ledit système d'imagerie étant connectés audit système de déclenchement, pour recevoir des signaux de déclenchement provenant du système de déclenchement, et étant commutés entre ledit état actif et un état non actif en fonction des signaux de déclenchement.

13. Procédé d'exécution de stroboscopie, incluant :
la génération de signaux de déclenchement à l'aide d'un procédé selon la revendication 11,
la génération d'une image desdites cordes vocales à partir de lumière réfléchie et/ou transmise par lesdites cordes vocales,
dans lequel ladite projection de lumière et ladite génération d'une image se font en fonction desdits signaux de déclenchement.

14. Laryngoscope incluant un système stroboscopique selon la revendication 12.
